# EUROPÄISCHE PATENTSCHRIFT

(11) **EP 0 363 318 B1**
(45) Veröffentlichungstag und Bekanntmachung des Hinweises auf die Patenterteilung: **02.03.1994**
(21) Anmeldenummer: 89810726.3
(22) Anmeldetag: 25.09.1989
(51) Int. Cl.: C07D 249/20

(54) **Verfahren zur Herstellung von Benztriazolderivaten**
Process for the preparation of benzotriazole derivatives
Procédé pour la préparation de dérivés de benzotriazole

(30) Priorität: 28.09.1988 CH 3594/88
(43) Veröffentlichungstag der Anmeldung: 11.04.1990
(73) Patentinhaber: CIBA-GEIGY AG, 4002 Basel (CH)
(72) Erfinder: Prestel, Helmut, Dr., D-7520 Bruchsal (DE); Müller, Klaus, Dr., D-6143 Lorsch (DE)

(56) Entgegenhaltungen:
- DE-A- 2 455 155
- GB-A- 1 494 824
- GB-A- 1 494 825
- US-A- 4 219 480
- US-A- 4 230 867
- CHEMICAL ABSTRACTS, Band 88, Nr. 15, 10 April 1978, Seite 575, Zusammenfassung Nr. 105346n, Columbus, Ohio, USA;

## Beschreibung

Die vorliegende Erfindung betrifft ein Verfahren zur Herstellung von halogenhaltigen 2-(2-Hydroxyphenyl)-2H-benztriazolen durch katalytische Hydrierung von entsprechenden o-Nitrophenylazohydroxyphenyl-Verbindungen in Gegenwart eines Pt-Hydrierkatalysators.

2-(2-Hydroxyphenyl)-2H-benztriazole sind als wertvolle UV-Absorber aus der Literatur bekannt. Sie werden in der Praxis in grossem Umfang als Lichtstabilisatoren für eine Vielzahl von Substraten eingesetzt, beispielsweise zur Stabilisierung von thermoplastischen Kunststoffen und von Ueberzugsmaterialien (z.B. von Lacken), aber auch in diversen Aufzeichnungsmaterialien (z.B. in photographischen Schichten und Papieren sowie in Drucktinten und Druckpapieren) und in Textilien.

Entsprechend der Bedeutung dieser Verbindungen wurde bereits eine ausserordentlich grosse Anzahl von Verfahren zu deren Herstellung vorgeschlagen. Ein Grossteil davon geht von den oben genannten o-Nitrophenylazo-Verbindungen aus und bedient sich der reduktiven Cyclisierung nach verschiedenen Reduktionsverfahren. Eines dieser Reduktionsverfahren ist die katalytische Hydrierung, die für die genannten Benztriazole in einer Reihe von Publikationen beschrieben wurde. Werden jedoch halogenhaltige o-Nitrophenylazo-Verbindungen hydriert, treten wegen Halogenabspaltung Probleme auf. Um diesen zu begegnen, konnte man nur bestimmte Hydrier-Katalysatoren verwenden oder es mussten zusätzliche Massnahmen ergriffen werden.

Die US-A 3,978,074 beschreibt ein in alkalischem und vorzugsweise in wässrigem Medium durchgeführtes Hydrierverfahren der vorstehend erwähnten Art, wobei als Hydrierkatalysatoren die üblichen Edelmetall- und andere Metallkatalysatoren eingesetzt werden. Es wird insbesondere darauf hingewiesen, dass bei Verwendung von Metallen (also z.B. Pt) als Katalysator Dehalogenierung von halogenhaltigen Ausgangsprodukten erfolgt. Als Abhilfe wird vorgeschlagen, Sulfidkatalysatoren (z.B. PtS, NiS usw.) einzusetzen, um Dehalogenierung zu vermeiden.

Gemäss GB-A 1 494 825 und 1 494 824 erfolgt die Hydrierung ebenfalls in alkalischem rein wässrigem (GB-A 1 494 825) oder wässrig/organischem (GB-A 1 494 824) Medium. Als Hydrierkatalysatoren werden Edelmetalle eingesetzt, wobei jedoch im Falle von chlorhaltigen Nitroazoverbindungen Pd nicht als Katalysator brauchbar ist. Zur Herstellung von chlorhaltigen Benztriazolen wird in beiden genannten GB-A als Katalysator jeweils Rh, insbesondere 5 % Rh auf Kohle, verwendet, woraus geschlossen werden kann, dass nicht nur Pd, sondern auch Pt als für die Herstellung von halogenhaltigen Produkten ungeeignet angesehen wurde.

Das in der GB-A 1 494 823 beschriebene Hydrierverfahren wird in organischen Lösungsmitteln unter Verwendung von organischen Aminen als Basen und der üblichen Edelmetallkatalysatoren durchgeführt, wobei im Falle von Chlorsubstitution Pd als Katalysator ausgeschlossen ist. Auch hier wird 5 % Rh auf Kohle im Fall von chlorsubstituierten Ausgangsprodukten eingesetzt (siehe Seite 3, linke Spalte, 2. Absatz; Beispiele 7 und 8).

Die US-A 4,219,480 lehrt die Verwendung eines Nickelkatalysators als Hydrierkatalysator. Beispiel 8 zeigt auch die Herstellung eines chlorhaltigen Benztriazols, allerdings mit einer nicht sehr hohen Ausbeute.

Die JP-A 52-113 973 befasst sich spezifisch mit der Herstellung von chlorsubstituierten 2-(2-Hydroxyphenyl)-2H-benztriazolen durch katalytische Hydrierung. Als Katalysator wird unter anderem auch 5 % Platin auf Kohle eingesetzt. Es zeigt sich jedoch, dass bei Verwendung von üblichen Basen wie NaOH und organischen Aminen (z.B. Triethylamin) die Ausbeuten relativ niedrig liegen (siehe Beispiele 6 und 7). Erst die Verwendung von unüblichen Basen wie NaBH₄ oder von sogenannten "Superbasen" wie z.B. 1,5-Diazabicyclo[5,4,0]undecen-5 führt zu etwas höheren Ausbeuten. Das gleiche Verfahren ist in der JP-A 52-113974 für nicht chlorsubstituierte Benztriazole beschrieben.

Es wurde nun überraschend gefunden, dass auch bei Verwendung von Pt als Hydrierkatalysator halogenierte (insbesondere chlorierte) 2-(2-Hydroxyphenyl)-2H-benztriazole ohne Dehalogenierung in hohen Ausbeuten erhalten werden können, auch bei Verwendung von einfachen Aminen als Basen. Dies ist dadurch möglich, dass man einen Pt-Katalysator auf einem Träger einsetzt, der das Pt in einer Menge von 0,1 - 3 Gew.-% enthält.

Das erfindungsgemässe Verfahren zur Herstellung von 2-(2-Hydroxyphenyl)-2H-benztriazolen der Formel 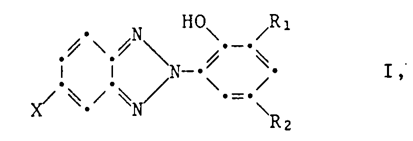
worin X Halogen, R₁ Wasserstoff, C₁-C₁₂-Alkyl, C₅-C₆-Cycloalkyl, Phenyl oder Phenyl-C₁-C₄-alkyl und R₂ C₁-C₁₂-Alkyl, C₅-C₆-Cycloalkyl, Phenyl, Phenyl-C₁-C₄-alkyl oder eine Gruppe -CₙH₂ₙ-COOR₃ bedeuten, worin n für 0 bis 4 und R₃ für Wasserstoff oder C₁-C₁₂-Alkyl stehen, durch katalytische Hydrierung einer Azoverbindung der Formel 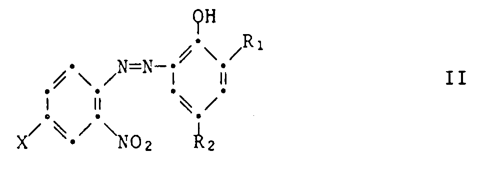
in Gegenwart eines Pt-Hydrierkatalysators und eines organischen Amins ist dadurch gekennzeichnet, dass man als Hydrierkatalysator Pt auf einem Träger verwendet, wobei die Belegung 0,1 bis 3 Gew.-% beträgt.

In Formel I bedeutet Halogen Chlor, Brom oder Fluor, insbesondere Chlor. Als Phenyl-C₁-C₄-alkyl (R₁, R₂) sind insbesondere Benzyl, Phenethyl, α-Methylbenzyl und α,α-Dimethylbenzyl, vorzugsweise Benzyl zu erwähnen. R₃ is vorzugsweise H oder C₁-C₄-Alkyl, insbesondere H oder Methyl. Aus den nieder-Alkylestern, insbesondere dem Methylester, können z.B. durch nachträgliche Verseifung Verbindungen mit R₃ = H oder durch Umesterung Verbindungen mit anderen Alkylgruppen R₃ hergestellt werden. Die Ausgangsverbindungen der Formel II sind bekannt, z.B. aus den eingangs angeführten Druckschriften oder aus der EP-A 57 160, oder können nach den dort angegebenen Verfahren hergestellt werden, etwa durch Diazotierung eines o-Nitroanilins der Formel 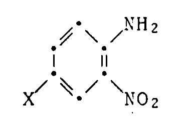
und Kupplung des resultierenden Diazoniumsalzes mit einem Phenol der Formel

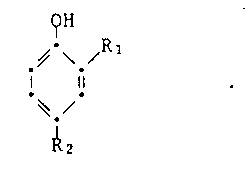

Bevorzugt werden Verbindungen der Formel I hergestellt, worin X Chlor ist, z.B. solche Verbindungen der Formel I, worin R₁ Wasserstoff, C₁-C₁₂-Alkyl oder Phenyl-C₁-C₃-alkyl und R₂ C₁-C₁₂-Alkyl, Phenyl-C₁-C₃-alkyl oder eine Gruppe -C₂H₄COOR₃ bedeuten, worin R₃ H oder C₁-C₁₂-Alkyl, insbesondere H oder C₁-C₄-Alkyl, bedeutet.

Von besonderer praktischer Bedeutung ist die Herstellung von Verbindungen der Formel I, worin R₁ Wasserstoff oder C₁-C₈-Alkyl und R₂ C₁-C₈-Alkyl bedeuten.

Erfindungswesentlich ist, dass im Pt-Katalysator das Pt in einer Menge (Belegung) von 0,1 - 3 Gew.-% enthalten ist. Vorteilhaft ist es, Katalysatoren zu verwenden, worin das Pt in einer Menge von 0,2 bis 2,5, insbesondere 0.3 - 2, z.B. 0.5 - 1,5, vorzugsweise 0,8 - 1,2 Gew. % vorhanden ist.

Als Träger kommen die in der Technik der Hydrierkatalysatoren üblichen in Frage, beispielsweise Kohle (z.B. Aktivkohle, Holzkohle, Torfkohle), Kieselgur, Aluminiumoxid, Bariumsulfat usw. Bevorzugt als Träger ist Kohle. Bevorzugte erfindungsgemässe Katalysatoren sind daher Pt/C-Katalysatoren, worin das Pt in den im vorstehenden Absatz angegebenen Mengen und bevorzugten Mengen enthalten ist.

Der Katalysator wird zweckmässig in einer Menge von 0,1 - 6 %, insbesondere 0,5 - 4 %, beispielsweise 1,0 - 3,0 %, bezogen auf die eingesetzte o-Nitroazoverbindung, eingesetzt. Der Katalysator ist selbstverständlich recyclierbar, zweckmässig durch Filtration, wenn das Verfahren batchwise (diskontinuierlich) durchgeführt wird.

Als organische Amine können aliphatische, cycloaliphatische oder aromatische Amine eingesetzt werden. Beispiele hierfür sind:
1) Mono-, Di- oder Tri-C₁-C₁₂-, insbesondere C₁-C₆-, vorzugsweise C₁-C₄-Alkylamine (wobei jede Alkylkette die angegebene C-Zahl enthalten kann und die einzelnen Alkylketten gleich oder verschieden sind), z.B. Methylamin, Ethylamin, n-Propylamin, i-Propylamin, n-Butylamin, i-Butylamin, t-Butylamin, Amylamin, Dimethylamin, Trimethylamin, Diethylamin, Triethylamin, Di-n-Propylamin, Di-i-Propylamin, Tri-n-Propylamin und Di-n-Butylamin.
2) Mono- oder Polyalkylenpolyamine. Beispiele dafür sind Verbindungen der Formel H₂N-CₙH₂ₙ(NH-CₘH₂ₘ)ₚ-NH₂, worin p eine Zahl von 0 bis 4 und n und m unabhängig voneinander eine Zahl von 1 bis 6 bedeuten, wobei im Fall p >1 die Indices m gleich oder verschieden sind. Bevorzugt handelt es sich bei den Alkylenketten um unverzweigte Reste. Im Fall von Polyalkylenpolyaminen (p ≠ 0) beträgt die Summe aller n + m vorzugsweise 2-20, insbesondere 4-18, z.B. 4-12. Besonders bevorzugt sind n und m unabhängig voneinander eine Zahl von 2 bis 4. Der Index p steht vorzugsweise für 0 (Alkylendiamine) oder für eine Zahl von 1 bis 3, vor allem 1 oder 2 (Polyalkylenpolyamine). Beispiele für Amine aus der Klasse der Monoalkylenpolyamine (Alkylendiamine) und Polyalkylenpolyamine sind die folgenden: Ethylendiamin, n-Propylendiamin, n-Butylendiamin, n-Pentylendiamin, n-Hexylendiamin, Diethylentriamin, Triethylentetramin, Di-n- oder i-propylentriamin, Tri-n- oder i-propylentetramin, Di-n-butylentriamin und Tri-n-butylentetramin.
3) Mono-, Di- und Tri-C₁-C₄-alkanolamine, wie z.B. Monoethanolamin, Diethanolamin, Triethanolamin, Mono-n-propanolamin, Di-n-propanolamin und Tri-n-propanolamin.
4) Cyclische Amine mit 5 - 7 C-Atomen, beispielsweise Cyclohexylamin, Methylcyclohexylamin und Cyclopentylamin.
5) Aromatische carbocyclische Amine, insbesondere Phenyl-, Diphenyl-, C₁-C₄-Alkylphenyl- und Phenyl-C₁-C₃-alkylamine, wie z.B. Anilin, o-, m-und p-Toluidin, o-, m- und p-Phenylendiamin, Benzylamin, Diphenylamin sowie N-C₁-C₄-Alkyl- und N,N-Di(C₁-C₄-alkyl)aniline wie N,N-Dimethylanilin.
6) Aromatische oder aliphatische heterocyclische Amine, insbesondere solche mit 5 oder insbesondere 6 Ringgliedern, wie z.B. Pyridin, Piperidin, Piperazin, N-Methylpiperazin und Morpholin.

Soweit heterocyclische Amine verwendet werden, sind diese vorzugsweise monocyclisch.

Bevorzugte Amine sind solche aus den obigen Gruppen 1), 2), 3) und 4), insbesondere 1), 2) und 3), vor allem 1) und 2). Besonders bevorzugt setzt man im erfindungsgemässen Verfahren Mono-, Di- oder Tri-(C₁-C₆alkyl-, insbesondere C₁-C₄-alkyl)-amine, oder Cyclohexylamin, vor allem Methylamin, Ethylamin, n-Propylamin, i-Propylamin, n-Butylamin, i-Butylamin, t-Butylamin, Cyclohexylamin, Dimethylamin, Diethylamin, Di-i-propylamin, Di-n-propylamin, Di-n-butylamin, Di-i-butylamin, Trimethylamin oder Triethylamin, sowie C₂-C₆-Alkylendiamine bzw. Polyalkylenpolyamine der Formel H₂N-(CH₂)_{n′}-[NH-(CH₂)_{m′}-[NH-(CH₂)_{p′}]_{q}-NH₂ mit n′, m′, p′ = 2 bis 4 (unabhängig voneinander) und q = 0 oder 1, insbesondere Ethylendiamin, n-Propylendiamin, n-Butylendiamin, n-Pentylendiamin, n-Hexylendiamin, Diethylentriamin, Triethylentetramin, Di-n-propylentriamin, Tri-n-propylentetramin, Di-n-butylentriamin und Tri-n-butylentetramin, vor allem Diethylentriamin, Triethylentetramin, Di-n-propylentriamin, Tri-n-propylentetramin, Ethylendiamin und n-Propylendiamin ein.

Besonders vorteilhaft als Base erweisen sich Mono-(C₁-C₄-alkyl)-amine wie i-Butylamin, n-Butylamin, n-Propylamin, i-Propylamin, Ethylamin und Methylamin, vor allem die ersten zwei genannten Alkylamine und Diethylamin, sowie C₂-C₆-Alkylendiamine bzw. Polyalkylenpolyamine wie Ethylendiamin, n-Propylendiamin, n-Butylendiamin, Diethylentriamin und Triethylentetramin.

Das Amin kann auch als Reaktionsmedium und einziges Lösungsmittel (besonders bei Verwendung von Mono-, Di- oder Tri-C₁-C₆-alkylaminen) fungieren. In diesem Fall wird kein weiteres Lösungsmittel für die Reaktanden und das Reaktionsprodukt benötigt.

Vorteilhaft ist jedoch im Reaktionsmedium mindestens ein weiteres Lösungsmittel vorhanden. Es kann sich dabei um Wasser oder um organische Lösungsmittel handeln. Wenn Verbindungen der Formel I hergestellt werden, in denen R₂ = -CₙH₂ₙ-COOR₃ und R₃ = H ist, ist dieses zusätzliche Lösungsmittel vorzugsweise Wasser, in allen anderen Fällen vorzugsweise ein organisches Lösungsmittel. Bei letzteren kann es sich um mit Wasser gut mischbare (polare) oder mit Wasser nicht oder schwer mischbare (unpolare) Lösungsmittel handeln. Wird nebem dem Amin ein organisches Lösungsmittel mitverwendet, ist dies vorzugsweise ein mit Wasser nicht oder schwer mischbares Lösungsmittel oder eine Kombination aus letzterem und einem mit Wasser gut mischbaren Lösungsmittel.

Beispiele für mit Wasser nicht oder schwer mischbare (unpolare) Lösungsmittel sind Kohlenwasserstoffe, wie etwa aliphatische Kohlenwasserstoffe (z.B. Hexan, Heptan, Petrolether); alicyclische Kohlenwasserstoffe (z.B. Cyclohexan, Methylcyclohexan) sowie aromatische Kohlenwasserstoffe (z.B. Benzol, Toluol, Xylol), aber auch halogenierte Kohlenwasserstoffe wie z.B. Chlorbenzol, Dichlorbenzol, Trichlorbenzol, Dichlorethan, Dichlorethylen, Trichlorethan usw.

Beispiele für mit Wasser gut mischbare (polare) Lösungsmittel sind Ether, Ester, Ketone, Amide (beispielsweise Diethylether, Dioxan, Tetrahydrofuran, Essigester, 1,2-Dimethoxyethan, 1,2-Diethoxyethan, Aceton, Ethylmethylketon, Diethylketon, Diisopropylketon, Formamid, Dimethylformamid usw.), Trialkylphosphate (z.B. Triethylphosphat), vor allem aber Alkohole, insbesondere niedrige aliphatische Alkohole (mit 1 - 6, insbesondere 1 - 4 C-Atomen), die z.B. 1-3-, insbesondere 1- oder 2-, vorzugsweise 1-wertig sind. Beispiele für solche Alkohole sind Methanol, Ethanol, n-Propanol, Isopropanol, sec-Butanol, n-Butanol, Amylalkohol, Hexanol, Ethylenglykol, Diethylenglykol, 2-Methoxyethanol.

Besonders bevorzugt wird im erfindungsgemässen Verfahren als Lösungsmittel ein aromatischer Kohlenwasserstoff, insbesondere Benzol, Toluol oder Xylol, oder eine Mischung der genannten Lösungsmittel mit einem niederen aliphatischen Alkohol, insbesondere n-Butanol oder i- oder n-Propanol, und vor allem Methanol oder Ethanol, eingesetzt.

Im Falle der Verwendung von Wasser als neben dem Amin verwendeten Lösungsmittel kann dieses allein oder in Kombination mit wassermischbaren oder/und mit Wasser nicht mischbaren organischen Lösungsmitteln eingesetzt werden. Beispiele und Bevorzugungen für solche organische Lösungsmittel sind vorstehend angegeben. Ein besonders bevorzugtes Lösungsmittelsystem für die Herstellung von Verbindungen der Formel I mit R₂ = -CₙH₂ₙ-COOR₃ und R₃ = H ist die Kombination von Wasser mit einem Mono- oder Di-C₁-C₄-Alkylamin, insbesondere von Wasser und Diethylamin, bevorzugt in einem Verhältnis von etwa 1:1.

Sofern das organische Amin nicht als Lösungsmittel fungiert, ist das Amin im Reaktionsgemisch zweckmässig in einer Menge von mindestens 0,1 Mol, insbesondere mindestens 0,5 Mol, vorzugsweise mindestens 0,9 Mol bis etwa 8 Mol pro Mol o-Nitroazobenzol-Ausgangsprodukt vorhanden. Uebernimmt das Amin teilweise oder ganz (als einziges Lösungsmittel) Löungsmittelfunktion, wird es mindestens in der für die Lösung oder Dispergierung der Reaktionskomponenten bzw. -produkte nötigen Menge eingesetzt.

Das erfindungsgemässe Verfahren kann diskontinuierlich (batchwise), aber auch kontinuierlich durchgeführt werden. Für die kontinuierliche Verfahrensweise eignet sich insbesondere ein Festbett-Katalysator, z.B. eine Hochdruck-Festbetthydrieranlage. Das Reaktionsgemisch wird in diesem Fall kontinuierlich abgezogen und mit frischer Nitroazoverbindung + Amin (ggf. + Lösungsmittel) gespeist.

Eine besonders vorteilhafte Variante des erfindungsgemässen Verfahrens, die eine kontinuierliche Arbeitsweise erlaubt und zu hohen Umsätzen und kurzen Reaktionszeiten führt, besteht darin, dass man in einem Autoklaven den Katalysator in einem Teil des Lösungsmittels vorlegt, den Autoklaven unter Wasserstoffdruck setzt und dann die entsprechende Verbindung der Formel II, gelöst oder dispergiert in einem weiteren Teil des Lösungsmittels, zudosiert, z.B. mit Hilfe einer Dosierpumpe. Die Reaktionslösung kann dann kontinuierlich abgezogen und daraus das Endprodukt in üblicher Weise isoliert werden. Alternativ kann auch in diskontinuierlicher Weise der Katalysator abfiltriert und das Filtrat entsprechend aufgearbeitet werden.

Die Hydrierung wird zweckmässig bei Temperaturen von 0 - 120°C, z.B. 15 - 100°C, insbesondere 20 - 80°C durchgeführt. Besonders vorteilhaft sind Reaktionstemperaturen von 25 - 50, insbesondere von 30 - 45°C.

Der Wasserstoffdruck kann bei der Hydrierung beispielsweise im Bereich von 1 - 100, beispielsweise 1 - 50, insbesondere 5 - 30, vorzugsweise 10 - 20 bar liegen. Welcher Wasserstoffdruck angewendet wird, hängt hauptsächlich von der zur Verfügung stehenden Hydrieranlage ab. Bei Hochdruckanlagen sind auch Drucke von 100 - 200 bar möglich. Solche sind vor allem bei kontinuierlicher Arbeitsweise üblich.

Die Hydrierzeit kann in weiten Grenzen schwanken, sie ist abhängig vom verwendeten Katalysator, vom Wasserstoffdruck, von der Reaktionstemperatur und der verwendeten Anlage. Sie kann z.B. von 30 Sekunden bis 5 Stunden betragen, insbesondere 10 Minuten bis 3 Stunden, z.B. 10 Minuten bis 2 Stunden. Bei kontinuierlicher Arbeitsweise ist z.B. in der Praxis mit Verweilzeiten von 1 bis 60 Minuten, insbesondere von 1 bis 30 Minuten zu rechnen.

Die Isolierung der Endprodukte aus dem Reaktionsmedium erfolgt nach üblichen, dem Fachmann bekannten Methoden. Sie variiert je nach der Art des verwendeten Lösungsmittels. Eine zweckmässige Methode besteht in der Ausfällung aus dem gegebenenfalls vorher eingeengten Reaktionsgemisch durch Zugabe eines Lösungsmittels, in dem das jeweilige Endprodukt schwer löslich ist und durch Abfiltrieren des Niederschlages. Aufarbeitung und allfällige Reinigungsoperationen sind auch den Beispielen zu entnehmen.

Wie bereits eingangs erwähnt, stellen die erfindungsgemäss herstellbaren 2-(2-Hydroxyphenyl)-2H-benztriazole wertvolle UV-Absorber dar, die in der Praxis als Lichtschutzmittel für eine Vielzahl von Anwendungen (wie beispielsweise in der Einleitung aufgezählt) eingesetzt werden können. Detaillierte Verwendungsmöglichkeiten der genannten Benztriazole sind in den US-A 3,055,896, 3,004,896, 3,072,585, 3,074,910, 3,189,615 und 3,230,194 beschrieben. Das erfindungsgemässe Verfahren eröffnet einen technisch besonders günstigen und wirtschaftlichen Weg zu deren Herstellung.

Die nachfolgenden Beispiele erläutern das erfindungsgemässe Verfahren weiter. Darin sowie in der übrigen Beschreibung und den Patentansprüchen bedeuten Teile Gewichtsteile und Prozentangaben Gewichtsprozent, sofern nichts anderes angegeben ist.

### Beispiel 1: 5-chlor-2-(2-hydroxy-3-tert.-butyl-5-methylphenyl)-2H-benztriazol

In einen 300 ml Hydrierreaktor gibt man bei Raumtemperatur unter Argon 60 g 2-Nitro-5-chlor-2′-hydroxy-3′-tert.butyl-5′-methylazobenzol (Gehalt 86 %), 40 g Xylol, 60 g n-Butylamin und 1 g 1 % Pt auf Aktivkohle. Anschliessend wird Argon durch Wasserstoff ersetzt. Nach Aufdrücken von 10 bar Wasserstoff wird unter intensiver Rührung bei 35 - 37°C hydriert. Durch Kühlung wird die freiwerdende Wärme abgeführt. Das Ende der Hydrierreaktion ist durch den Stillstand der Wasserstoffaufnahme nach 2 Moläquivalenten Wasserstoff, bezogen auf das Azobenzol-Ausgangsprodukt, leicht zu erkennen. Die gesamte Hydrierzeit beträgt ca. 1,5 Stunden.

Nach Erhitzen der Reaktionsmasse auf 70 - 80°C wird der Katalysator durch Filtration abgetrennt. Durch Destillation wird das n-Butylamin und ein Teil des Xylols aus dem Filtrat entfernt. Anschliessend versetzt man die resultierende xylolische Lösung mit 150 g Methanol und fällt somit das gewünschte Produkt aus. Nach Abkühlen der Suspension auf 0°C filtriert und trocknet man die Kristalle (Titelverbindung) ab. Ausbeute 43,3 g, entsprechend 91 % der Theorie. Schmelzpunkt 138 - 140°C.

### Beispiel 2:

Beispiel 1 wird wiederholt, wobei jedoch an Stelle von 60 g n-Butylamin und 40 g Xylol 100 g n-Butylamin eingesetzt werden. Es ist kein Einfluss auf die Hydriergeschwindigkeit bzw. Ausbeute zu beobachten. Ebenso zeigt ein Ersatz von Xylol durch Toluol oder Benzol keinen negativen Effekt.

### Beispiel 3:

Die Beispiele 1 und 2 werden wiederholt, wobei jedoch der Hydrierdruck von 10 auf 20 bar Wasserstoff erhöht wird. Die Hydrierzeit wird von ca. 1,5 Stunden auf ca. 1 Stunde verkürzt. Die Aufarbeitung ist analog, man erhält praktisch die gleiche Ausbeute.

### Beispiel 4: 5-Chlor-2-(2-hydroxy-3,5-di-tert-butylphenyl)-2H-benztriazol

Die Beispiele 1 und 2 werden wiederholt, wobei jedoch an Stelle des 2-Nitro-5-chlor-2′-hydroxy-3′-tert.butyl-5′-methylazobenzols eine äquivalente Menge an 2-Nitro-5-chlor-2′hydroxy-3′,5′-di-tert.-butylazobenzol (Gehalt 91 %) eingesetzt wird. Das Titelprodukt wird in einer Ausbeute von 46,1 g (92 % der Theorie) isoliert; Schmelzpunkt 154 - 157°C.

### Beispiel 5:

Wird Beispiel 4 in der Variante gemäss Beispiel 1 wiederholt, jedoch n-Butylamin durch die äquivalente Menge Diethylamin ersetzt, wird die Verbindung des Beispiels 4 in ähnlicher Ausbeute erhalten.

### Beispiel 6:

Beispiel 4 in der Variante nach Beispiel 1 wird wiederholt, wobei jedoch die Hydriertemperatur von 35 - 37°C auf 60°C erhöht wird. Die Hydrierzeit von ca. 1,5 Stunden wird dadurch auf ca. 0,5 Stunden verkürzt. Man isoliert das Produkt in einer Ausbeute von 43,6 g (87 % der Theorie).

### Beispiel 7:

Beispiel 4 in der Variante gemäss Beispiel 1 wird wiederholt, wobei jedoch an Stelle von 1 % Pt auf Aktivkohle die gleiche Menge von 0,5 % Pt auf Aktivkohle als Katalysator eingesetzt wird. Das Produkt wird dann in einer Ausbeute von 93 % der Theorie erhalten.

### Beispiel 8:

Beispiel 4 in der Variante gemäss Beispiel 1 wird wiederholt, wobei jedoch an Stelle von 1 % Pt auf Aktivkohle die gleiche Menge von 2 % Pt auf Aktivkohle als Katalysator eingesetzt wird. Das Produkt wird dann in einer Ausbeute von 86 % der Theorie erhalten.

### Beispiel 9:

In einen 2 l-Hydrierreaktor gibt man bei Raumtemperatur unter Argon 200 g n-Butylamin und 5,5 g 1 % Pt auf Aktivkohle. Der Reaktor wird verschlossen und Argon durch Wasserstoff ersetzt. Nach Aufdrücken von 10 bar Wasserstoff wird der Katalysator im n-Butylamin durch intensive Rührung dispergiert. Gleichzeitig werden in einem externen Behälter 300 g 2-Nitro-5-chlor-2′-hydroxy-3′,5′-di-tert.-butylazobenzol (Gehalt 91 %) in 300 g Xylol bei Raumtemperatur dispergiert. Diese Dispersion wird mit einer automatischen Dosiereinrichtung über einen Zeitraum von 1 Stunde gegen den Wasserstoffdruck in den Hydrierreaktor gepumpt. Dabei findet die Hydrierung der Azoverbindung bei 20 - 40°C zu dem entsprechenden Benztriazol statt.

Nach Beendigung der Dosierung wird die Reaktionsmasse auf 70 - 80°C erhitzt und der Katalysator durch Filtration abgetrennt. Die weitere Aufarbeitung erfolgt analog Beispiel 1 mit den entsprechend erhöhten Lösungsmittelmengen. Man erhält so 228 g (91 % der Theorie) 5-Chlor-2-(2-hydroxy-3,5-di-tert-butylphenyl)-2H-benztriazol mit einem Schmelzpunkt von 154 - 157°C.

Eine Erhöhung der Hydriertemperatur von 20 - 40°C auf 50°C oder eine Erhöhung des Hydrierdruckes von 10 auf 20 bar oder eine Verkürzung (Verlängerung) der Dosierzeit der Azoverbindung von 1 Stunde auf 5 Minuten (2 Stunden) hat keinen signifikanten Einfluss auf die Produktausbeute. Man erhält praktisch die gleiche Ausbeute.

### Beispiel 10: 5-Chlor-2-[2-hydroxy-3-tert-butyl-5-(2-methoxycarbonylethyl)-phenyl]-2H-benztriazol

In einen 1 l-Hydrierreaktor gibt man bei Raumtemperatur unter Argon 93 g 2-Nitro-5-chlor-2′-hydroxy-3′-tert-butyl-5′-carboxyethylazobenzol (Gehalt 88 %), 108 g Wasser, 108 g Diethylamin und 1,9 g 1 % Pt auf Aktivkohle.

Anschliessend wird Argon durch Wasserstoff ersetzt. Nach Aufdrücken von 10 bar Wasserstoff wird unter intensiver Rührung bei 40°C hydriert. Die gesamte Hydrierzeit beträgt ca. 1 Stunde. Das Ende der Hydrierreaktion ist durch den Stillstand der Wasserstoffaufnahme gekennzeichnet.

Nach Erhitzen der Reaktionsmasse auf 60°C wird der Katalysator durch Filtration abgetrennt. Durch Destillation wird das Diethylamin und ein Teil des Wassers aus dem Filtrat entfernt. Anschliessend gibt man 500 g Xylol zu und überführt die Benztriazolcarbonsäure durch Ansäuern mit ca. 15 g Schwefelsäure (83%-ig) in die organische Phase. Nach Abtrennen der wässrigen Unterphase wird die xylolische Phase destillativ aufkonzentriert und die Benztriazolcarbonsäure (bei ca. 70°C) durch Zugabe von 280 g Methanol und 10 g Schwefelsäure (konz.) verestert; dabei fällt das Produkt (Titelverbindung) aus. Die Kristalle werden abgetrennt und getrocknet. (Ausbeute 71,2 g, entsprechend 91 % der Theorie). Schmelzpunkt: 125 - 128°C
Das durch die Hydrierung entstehende 5-Chlor-2-[2-hydroxy-3-tert-butyl-5-(2-carboxyethyl)phenyl]-2H-benztriazol kann aus dem Reaktionsgemisch, falls gewünscht, auch als solches isoliert werden, z.B. durch Ansäuern der Reaktionsmischung nach beendeter Hydrierung und Filtration des ausgefallenen Produktes.

### Beispiel 11: 5-Chlor-2-(2-hydroxy-3,5-di-tert.-butylphenyl)-2H-benztriazol

Beispiel 4 in der Variante gemäss Beispiel 1 wird wiederholt, wobei an Stelle von 60 g n-Butylamin und 40 g Xylol eine Mischung aus 40 g Diethylentriamin und 60 g Xylol eingesetzt werden. Die gesamte Hydrierzeit beträgt 2 Stunden.

Nach der Katalysatorfiltration ist die Reaktionslösung zweiphasig (schwarze, aminreiche Unterphase und eine xylolische, das Produkt enthaltende Oberphase). Die aminreiche Unterphase kann zweckmässigerweise zur besseren Aufarbeitung und Wiederverwendung des Diethylentriamins abgetrennt oder aber im System belassen werden.

Durch die Zugabe von 20 g Wasser zur Reaktionslösung und anschliessende Phasentrennung der wässrigen, aminischen Unterphase bei ca. 80-90°C kann das Diethylentriamin praktisch quantitativ aus der Reaktionslösung entfernt werden.

Nach der üblichen Aufarbeitung entsprechend Beispiel 1 wird die Titelverbindung in einer Ausbeute von 95 % der Theorie isoliert.

Eine Erhöhung des Hydrierdruckes von 10 auf 50 bar zeigt ausser einer Verkürzung der Hydrierzeit keinen weiteren Effekt. Man erhält praktisch die gleiche Ausbeute.

Die Verwendung einer äquivalenten Menge Ethylendiamin oder Triethylentetramin an Stelle von 40 g Diethylentriamin zeigt keinen signifikanten Einfluss weder auf den Hydrierverlauf noch auf die erhaltene Produktausbeute.

### Beispiel 12:

Wird Beispiel 4 in der Variante gemäss Beispiel 1 wiederholt, jedoch an Stelle von 60 g n-Butylamin und 40 g Xylol entweder 40 g Monoethanolamin und 60 g Xylol oder 40 g Cyclohexylamin und 60 g Xylol eingesetzt, wird die Verbindung des Beispiels 4 in etwas geringerer Ausbeute wie in Beispiel 4 angegeben erhalten.

### Beispiel 13:

Beispiel 4 in der Variante gemäss Beispiel 1 wird wiederholt, wobei an Stelle von 40 g Xylol eine Mischung aus 30 g Xylol mit 10 g Methanol eingesetzt wird. Die Verbindung des Beispiels 4 wird in einer Ausbeute von 87 % der Theorie isoliert.

### Beispiel 14:

Beispiel 1 wird wiederholt, wobei jedoch an Stelle von 60 g n-Butylamin und 40 g Xylol eine Mischung aus 40 g Diethylentriamin und 60 g Xylol eingesetzt wird. Nach der Aufarbeitung der Reaktionsmasse wie in Beispiel 1 angegeben wird das Reaktionsprodukt aus Xylol/Methanol kristallisiert. Man erhält die Verbindung des Beispiels 1 in einer Ausbeute von 92 % der Theorie.

## Patentansprüche

1. Verfahren zur Herstellung von 2-(2-Hydroxyphenyl)-2H-benztriazolen der Formel 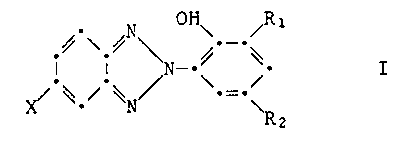 worin X Halogen, R₁ Wasserstoff, C₁-C₁₂-Alkyl, C₅-C₆-Cycloalkyl, Phenyl oder Phenyl-C₁-C₄-alkyl und R₂ C₁-C₁₂-Alkyl, C₅-C₆-Cycloalkyl, Phenyl, Phenyl-C₁-C₄-alkyl oder eine Gruppe -CₙH₂ₙ-COOR₃ bedeuten, worin n für 0 bis 4 und R₃ für Wasserstoff oder C₁-C₁₂-Alkyl stehen, durch katalytische Hydrierung einer Azoverbindung der Formel 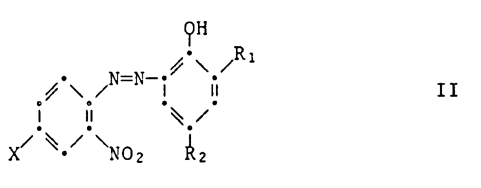 in Gegenwart eines Pt-Hydrierkatalysators und eines organischen Amins, dadurch gekennzeichnet, dass man als Hydrierkatalysator Pt auf einem Träger verwendet, wobei die Pt-Belegung 0,1 bis 3 Gew.-% beträgt.

2. Verfahren nach Anspruch 1, worin X Chlor bedeutet.

3. Verfahren nach Anspruch 1 oder 2, worin R₁ Wasserstoff, C₁-C₁₂-Alkyl oder Phenyl-C₁-C₃-alkyl und R₂ C₁-C₁₂-Alkyl, Phenyl-C₁-C₃-alkyl oder eine Gruppe -C₂H₄COOR₃ bedeuten, worin R₃ Wasserstoff oder C₁-C₁₂-Alkyl, insbesondere Wasserstoff oder C₁-C₄-Alkyl bedeutet.

4. Verfahren nach Anspruch 3, worin R₁ Wasserstoff oder C₁-C₈-Alkyl und R₂ C₁-C₈-Alkyl bedeuten.

5. Verfahren nach einem der Ansprüche 1 - 4, worin als Katalysator 0,3 bis 2 % Pt auf Kohle verwendet wird.

6. Verfahren nach Anspruch 5, worin als Katalysator 0,5 - 1,5 % Pt auf Kohle verwendet wird.

7. Verfahren nach einem der Ansprüche 1 - 6, worin als organisches Amin ein Mono-, Di- oder Tri-C₁-C₆-Alkylamin, Cyclohexylamin oder ein C₂-C₆-Alkylendiamin oder ein Polyalkylenpolyamin der Formel H₂N-(CH₂)_{n′}-NH-(CH₂)_{m′}-[NH-(CH₂)_{p′}]_{q}-NH₂, worin n′, m′ und p′ unabhängig voneinander 2 bis 4 und q 0 oder 1 bedeuten, eingesetzt wird.

8. Verfahren nach Anspruch 7, worin das Amin Methylamin, Ethylamin, n-Propylamin, i-Propylamin, n-Butylamin, i-Butylamin, t-Butylamin, Cyclohexylamin, Dimethylamin, Diethylamin, Di-n-propylamin, Di-i-propylamin, Di-n-butylamin, Trimethylamin, Triethylamin, Ethylendiamin, n-Propylendiamin, n-Butylendiamin, n-Pentylendiamin, n-Hexylendiamin, Diethylentriamin, Triethylentetramin, Di-n-propylentriamin, Tri-n-propylentetramin, Di-n-butylentriamin oder Tri-n-butylentetramin ist.

9. Verfahren nach Anspruch 8, worin das Amin Diethylamin, i-Butylamin, n-Butylamin, Ethylendiamin, n-Propylendiamin, Diethylentriamin oder Triethylentetramin ist.

10. Verfahren nach einem der Ansprüche 1 - 9, worin das organische Amin als einziges Lösungsmittel fungiert.

11. Verfahren nach einem der Ansprüche 1 - 9, worin man der Reaktionsmischung zusätzlich ein mit Wasser nicht oder schwer mischbares organisches Lösungsmittel zusetzt.

12. Verfahren nach Anspruch 11, worin das organische Lösungsmittel ein Kohlenwasserstoff, insbesondere ein aromatischer Kohlenwasserstoff ist.

13. Verfahren nach Anspruch 12, worin das organische Lösungsmittel Benzol, Toluol oder Xylol ist.

14. Verfahren nach einem der Ansprüche 1 - 9, worin man im Fall R₂ = -CₙH₂ₙ-COOR₃ und R₃ = H zusätzlich Wasser als Lösungsmittel zusetzt.

15. Verfahren nach Anspruch 11 oder 14, worin man der Reaktionsmischung zusätzlich ein mit Wasser mischbares organisches Lösungsmittel, insbesondere einen niederen aliphatischen Alkohol, zusetzt.

16. Verfahren nach Anspruch 14 oder 15, worin man der Reaktionsmischung zusätzlich ein mit Wasser nicht mischbares organisches Lösungsmittel zusetzt.

## Claims

1. A process for the preparation of a 2-(2-hydroxyphenyl)-2H-benzotriazole of the formula 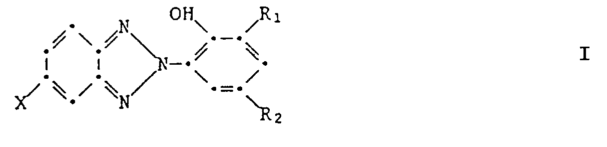 in which X is halogen, R₁ is hydrogen, C₁-C₁₂alkyl, C₅-C₆cycloalkyl, phenyl or phenyl-C₁-C₄alkyl and R₂ is C₁-C₁₂alkyl, C₅-C₆cycloalkyl, phenyl, phenyl-C₁-C₄alkyl or a group -CₙH₂ₙ-COOR₃, in which n is 0 to 4 and R₃ is hydrogen or C₁-C₁₂alkyl, by catalytic hydrogenation of an azo compound of the formula 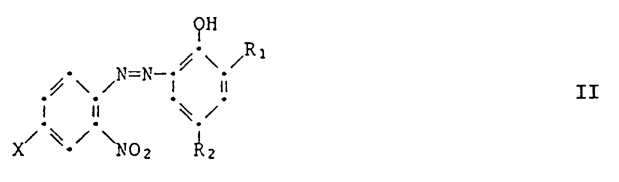 in the presence of a Pt hydrogenation catalyst and an organic amine, wherein the hydrogenation catalyst used is Pt on a support, the Pt applied being 0.1 to 3 % by weight.

2. A process according to claim 1, wherein X is chlorine.

3. A process according to either of claims 1 or 2, wherein R₁ is hydrogen, C₁-C₁₂alkyl or phenyl-C₁-C₃alkyl and R₂ is C₁-C₁₂alkyl, phenyl-C₁-C₃alkyl or a group -C₂H₄COOR₃, in which R₃ is hydrogen or C₁-C₁₂alkyl, especially hydrogen or C₁-C₄alkyl.

4. A process according to claim 3, wherein R₁ is hydrogen or C₁-C₈alkyl and R₂ is C₁-C₈alkyl.

5. A process according to any one of claims 1-4, wherein 0.3 to 2 % Pt on carbon is used as the catalyst.

6. A process according to claim 5, wherein 0.5-1.5 % Pt on carbon is used as the catalyst.

7. A process according to any one of claims 1-6, wherein the organic amine used is a mono-, di- or tri-C₁-C₆alkylamine, cyclohexylamine or a C₂-C₆alkylenediamine or a polyalkylenepolyamine of the formula H₂N-(CH₂)_{n'}-NH-(CH₂)_{m'}-[NH-(CH₂)_{p'}]_{q}-NH₂ in which n', m' and p' independently of one another are 2 to 4 and q is 0 or 1.

8. A process according to claim 7, wherein the amine is methylamine, ethylamine, n-propylamine, i-propylamine, n-butylamine, i-butylamine, t-butylamine, cyclohexylamine, dimethylamine, diethylamine, di-n-propylamine, di-i-propylamine, di-n-butylamine, trimethylamine, triethylamine, ethylenediamine, n-propylenediamine, n-butylenediamine, n-pentylenediamine, n-hexylenediamine, diethylenetriamine, triethylenetetramine, di-n-propylenetriamine, tri-n-propylenetetramine, di-n-butylenetriamine or tri-n-butylenetetramine.

9. A process according to claim 8, wherein the amine is diethylamine, i-butylamine, n-butylamine, ethylenediamine, n-propylenediamine, diethylenetriamine or triethylenetetramine.

10. A process according to any one of claims 1-9, wherein the organic amine functions as the sole solvent.

11. A process according to any one of claims 1-9, wherein an organic solvent which is immiscible or sparingly miscible with water is additionally added to the reaction mixture.

12. A process according to claim 11, wherein the organic solvent is a hydrocarbon, especially an aromatic hydrocarbon.

13. A process according to claim 12, wherein the organic solvent is benzene, toluene or xylene.

14. A process according to any one of claims 1-9, wherein, in the case of R₂ = -CₙH₂ₙ-COOR₃ and R₃ = H, water is added additionally as a solvent.

15. A process according to either of claims 11 or 14, wherein a water-miscible organic solvent, especially a lower aliphatic alcohol, is added additionally to the reaction mixture.

16. A process according to either of claims 14 or 15, wherein a water-immiscible organic solvent is added additionally to the reaction mixture.

## Revendications

1. Procédé de préparation de 2-(2-hydroxyphényl)-2H-benzotriazoles de formule 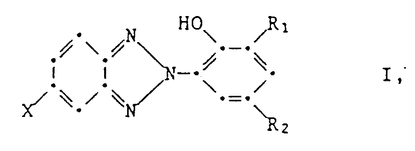 dans laquelle X représente un halogène, R₁ représente l'hydrogène, un alkyle en C₁-C₁₂, un cycloalkyle en C₅-C₆, un phényle ou un phényl-(alkyle en C₁-C₄) et R₂ représente un alkyle en C₁-C₁₂, un cycloalkyle en C₅-C₆, un phényle, un phényl-(alkyle en C₁-C₄) ou un groupe -CₙH₂ₙ-COOR₃ où n est 0 à 4 et R₃ représente l'hydrogène ou un alkyle en C₁-C₁₂, par hydrogénation catalytique d'un composé azoïque de formule 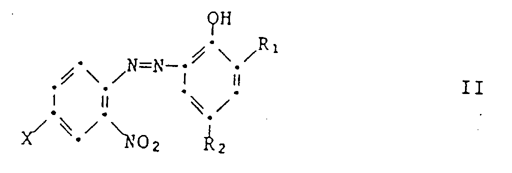 en présence d'un catalyseur d'hydrogénation au Pt et d'une amine organique, caractérisé en ce que l'on utilise comme catalyseur d'hydrogénation du Pt sur un support, le revêtement de Pt étant de 0,1 à 3 % en masse.

2. Procédé selon la revendication 1, dans lequel X représente le chlore.

3. Procédé selon la revendication 1 ou 2, dans lequel R₁ représente l'hydrogène, un alkyle en C₁-C₁₂ ou un phényl-(alkyle en C₁-C₃) et R₂ représente un alkyle en C₁-C₁₂, un phényl-(alkyle en C₁-C₃) ou un groupe -C₂H₄COOR₃ où R₃ représente l'hydrogène ou un alkyle en C₁-C₁₂, en particulier l'hydrogène ou un alkyle en C₁-C₄.

4. Procédé selon la revendication 3, dans lequel R₁ représente l'hydrogène ou un alkyle en C₁-C₈ et R₂ représente un alkyle en C₁-C₈.

5. Procédé selon l'une des revendications 1 à 4, dans lequel on utilise comme catalyseur du Pt à 0,3 à 2 % sur du charbon.

6. Procédé selon la revendication 5, dans lequel on utilise comme catalyseur du Pt à 0,5 - 1,5 % sur du charbon.

7. Procédé selon l'une des revendications 1 à 6, dans lequel on utilise comme amine organique une mono-, di- ou tri-alkyl en C₁-C₆-amine, la cyclohexylamine, ou une alkylènediamine en C₂-C₆ ou une polyalkylènepolyamine de formule H₂N-(CH₂)_{n'}-NH-(CH₂)_{m'}-[NH-(CH₂)_{p'}]_{q}-NH₂ où n', m', p' sont, indépendamment les uns des autres, 2 à 4, et q est 0 ou 1.

8. Procédé selon la revendication 7, dans lequel l'amine est la méthylamine, l'éthylamine, la n-propylamine, l'i-propylamine, la n-butylamine, l'i-butylamine, la t-butylamine, la cyclohexylamine, la diméthylamine, la diéthylamine, la di-n-propylamine, la di-i-propylamine, la di-n-butylamine, la triméthylamine, la triéthylamine, l'éthylènediamine, la n-propylènediamine, la n-butylènediamine, la n-pentylènediamine, la n-hexylènediamine, la diéthylènetriamine, la triéthylènetétramine, la di-n-propylènetriamine, la tri-n-propylènetétramine, la di-n-butylènetriamine ou la tri-n-butylènetétramine.

9. Procédé selon la revendication 8, dans lequel l'amine est la diéthylamine, l'i-butylamine, la n-butylamine, l'éthylènediamine, la n-propylènediamine, la diéthylènetriamine ou la triéthylènetétramine.

10. Procédé selon l'une des revendications 1 à 9, dans lequel l'amine organique fait fonction de solvant unique.

11. Procédé selon l'une des revendications 1 à 9, dans lequel on ajoute en plus au mélange réactionnel un solvant organique non miscible à l'eau ou difficilement miscible à l'eau.

12. Procédé selon la revendication 11, dans lequel le solvant organique est un hydrocarbure, en particulier un hydrocarbure aromatique.

13. Procédé selon la revendication 12, dans lequel le solvant organique est le benzène, le toluène ou le xylène.

14. Procédé selon l'une des revendications 1 à 9, dans lequel, au cas où R₂= -CₙH₂ₙ-COOR₃ et R₃ = H, on ajoute en plus de l'eau comme solvant.

15. Procédé selon la revendication 11 ou 14, dans lequel on ajoute en plus au mélange réactionnel un solvant organique miscible à l'eau, en particulier un alcool aliphatique inférieur.

16. Procédé selon la revendication 14 ou 15, dans lequel on ajoute en plus au mélange réactionnel un solvant organique non miscible à l'eau.
